# EUROPEAN PATENT SPECIFICATION

(11) **EP 4 262 637 B1**
(45) Date of publication and mention of the grant of the patent: **23.04.2025**
(21) Application number: 21844962.7
(22) Date of filing: 21.12.2021
(51) Int. Cl.: A61F 2/46

(54) **IMPROVEMENTS IN AND RELATING TO SURGICAL INSTRUMENTS AND SYSTEMS**
VERBESSERUNGEN AN UND IM ZUSAMMENHANG MIT CHIRURGISCHEN INSTRUMENTEN UND SYSTEMEN
PERFECTIONNEMENTS APPORTÉS OU SE RAPPORTANT À DES MÉTHODES ET SYSTÈMES CHIRURGICAUX

(30) Priority: 21.12.2020 GB 202020305
(43) Date of publication of application: 25.10.2023
(73) Proprietor: DePuy Ireland Unlimited Company, County Cork (IE)
(72) Inventor: BIRKBECK, Alec, Leeds Yorkshire LS11 8DT (GB); FONSECA, Jeremy, Leeds Yorkshire LS11 8DT (GB); SHAPLAND, John, Leeds Yorkshire LS11 8DT (GB); GOSLING, Niki, Leeds Yorkshire LS11 8DT (GB)
(74) Representative: Murgitroyd & Company
(86) International application number: PCT/EP2021/086965
(87) International publication number: WO 2022/136359

(56) References cited:
- EP-A1- 2 740 418
- US-A- 6 063 123
- US-A1- 2014 228 854
- US-A1- 2019 388 243

## Description

### TECHNICAL FIELD

The disclosure is concerned with the removal of a liner from an acetabular shell implant, for instance of the type used in hip arthroplasty. The disclosure includes surgical instruments for the removal of such liners, systems for the removal of such liners and methods of use for the surgical instruments and/or systems. The closest prior art is document US 6488713 A, which defines the preamble of claim 1.

### BACKGROUND

In hip arthroplasty, the anatomic reconstruction of the joint is sought. The natural acetabulum is removed and replaced with an implant formed of an acetabular shell placed in the recess in the bone and fixed in position. Subsequently, an appropriately sized liner is inserted into the shell, mated and locked. On occasions, it is necessary to remove a polyethylene acetabular liner from an implanted shell, for instance to remove that liner and replace it with another.

Existing designs provide for jaw based surgical instruments which are used to take hold of the liner and apply a force to pull the liner from the shell.

It is desirable for the surgical instrument, system or methods of use to remove the liner without damaging or risking damage to the shell holding it. It is desirable for the surgical instrument, system or methods of use to be easily positioned, used and removed. It is desirable for the surgical instrument, system or method of use to effectively and reliably remove the liner, in wound sites which are hard to access or have limited access.

### SUMMARY

A surgical instrument according to the invention is defined in claim 1, with various embodiments defined in dependent claims 2-6.

According to a second aspect of the invention there is provided a surgical system according to claim 7 and its dependent claims.

### BRIEF DESCRIPTION OF THE DRAWINGS

Various embodiments of the disclosure will now be described, by way of example only, and with reference to the accompanying drawings in which:
Figure 1a is a side view of a prior art liner extraction instrument;
Figure 1b is a perspective view of the instrument of Figure 1a engaging a liner;
Figure 1c is a side view corresponding to Figure 1b;
Figure 1d is a side view with the liner partially removed from the shell;
Figure 2 is a perspective view of a system, including a guide instrument, drill bit, screw and drive extension;
Figure 3 is a superior plan view of the distal end of the guide instrument and sectioned drill bit engaged with a liner in a shell;
Figure 4 is a side view corresponding to Figure 3;
Figure 5 is a perspective view of details of a distal end of the drive extension, proximal end of a drill bit and proximal end of a screw;
Figure 6 is a side view showing the interaction with a first liner and shell combination;
Figure 7 is a side view showing the interaction with a second liner and shell combination;
Figure 8 is a detailed side perspective view of the guide piece with a drill bit present; and
Figure 9 shows the pilot hole relative to a liner and shell.

### DETAILED DESCRIPTION OF THE DRAWINGS

In hip arthroplasty the anatomic reconstruction of the joint is sought. The natural acetabulum is removed and replaced with an implant formed of an acetabular shell placed in the recess in the bone and fixed in position. Subsequently, an appropriate sized liner is inserted into the shell, mated and locked. On occasions, it is necessary to remove a polyethylene acetabular liner from an implanted shell, for instance to remove that liner and replace it with another. This must be done whilst minimising the risk of damage to the shell holding it.

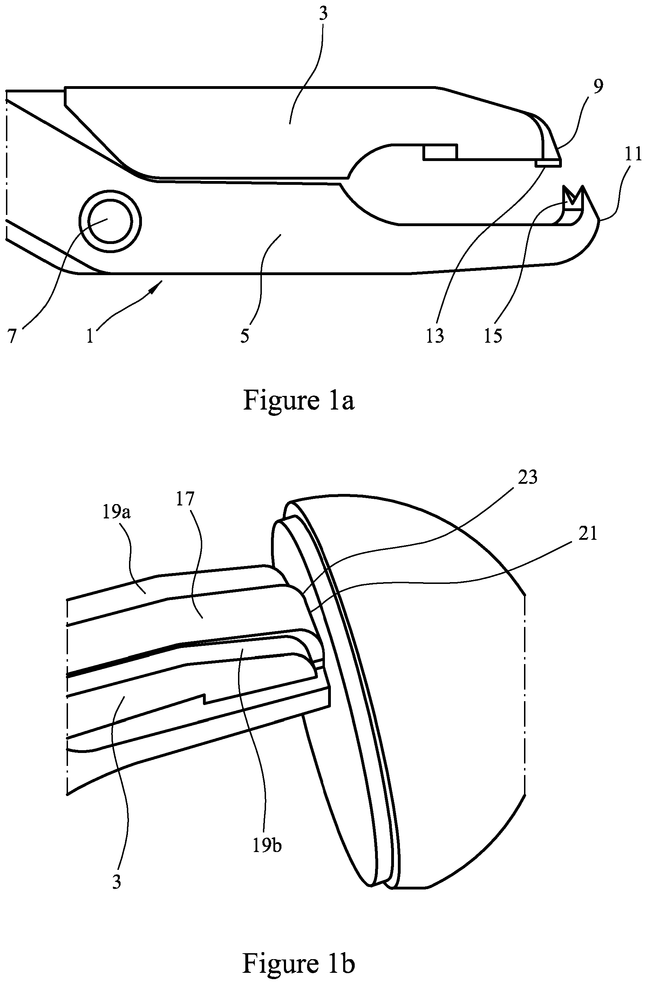

One prior art instrument, a liner extractor 1, is illustrated in Figure 1a. This provides a first jaw 3 and second jaw 5 pivotally mounted relative to one another about pin 7. The distal end 9 of the first jaw 3 extends beyond the distal end 11 of the second jaw 5. The distal end 9 of the first jaw 3 is provided with a pair of contact surfaces 13 facing the second jaw 5. The distal end 11 of the second jaw 5 is provided with a pair of teeth 15 facing generally towards the first jaw 3.

As can be seen in Figure 1b, the first jaw 3 has a separate central element 17 which is provided between a first jaw element 19a and second jaw element 19b which in combination form the first jaw 3. The distal end 21 of the central element 17 has an abutment surface 23 generally perpendicular to the pair of contact surfaces 13.

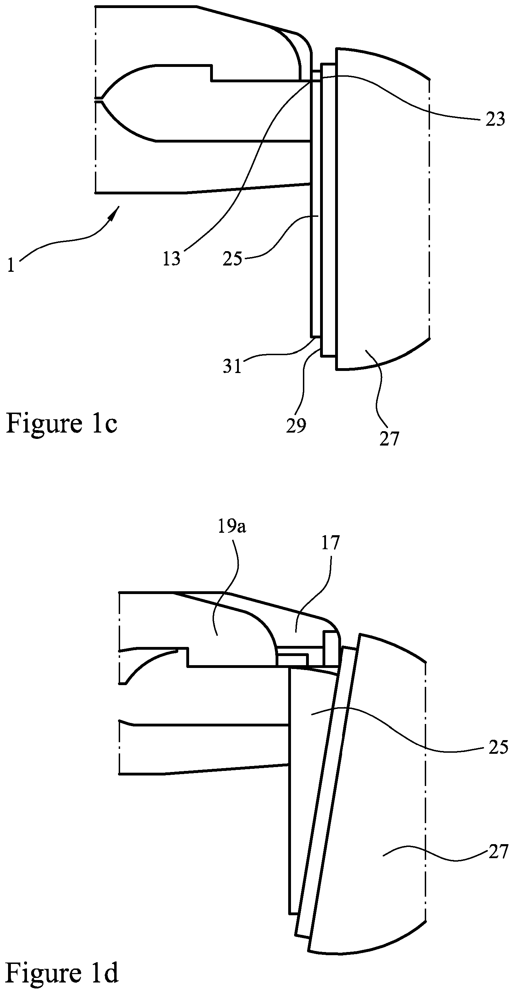

With the first jaw 3 and second jaw 5 open, wider than is shown in Figure 1a where the closed state is shown, the liner extractor 1 can be engaged with a liner 25 sitting in a shell 27, as shown in Figure 1c. The abutment surface 23 is brought into contact with the circumferential face 29 of the shell 27. This limits axial movement of the liner extractor 1 relative to the shell 27. The pair of contact surfaces 13 are brought into abutment with the small side wall 31 of the liner 25. Closing the jaws, by moving the second jaw 5 inwards towards the first jaw 3, brings the pair of teeth 15 into contact with the inside of the liner 25. The teeth 15 are sharp enough to penetrate the liner 25 upon application of force through the liner extractor 1, as the contact surfaces 13 and side wall 31 combine to prevent movement of the first jaw 3.

In Figure 1d, the partial removal of the liner 25 from the shell 27 is shown. This is achieved by retraction of the first jaw element 19a and second jaw element 19b relative to the central element 17. Hence, the central element 17 maintains the position of the shell 27 and the liner 25 is eased out of the shell 27.

As access to the wound site is not easy, the positioning of the liner extractor 1 with the abutment surface 23 and contact surfaces 13 in the necessary position during movement of the contact surfaces and abutment surface relative to one another can be awkward.

Another instrument-based option for liner 25 removal is to form a pilot hole in the liner 25. Initial engagement of a self-tapping screw in the pilot hole is then provided, with tightened of the screw pushing between the liner 25 and the shell 27 with sufficient force to remove the liner 25 from the shell 27. Where the liner 25 is of the thicker and/or larger and/or stiffer variety, then multiple pilot holes and self-tapping screws may be needed.

In this type of option, to be effective, the pilot hole needs to be created in a consistent and precise location and also with a consistent angularity with respect to the liner 25. This needs to be achieved in a wound space which frequently only offers difficult access.

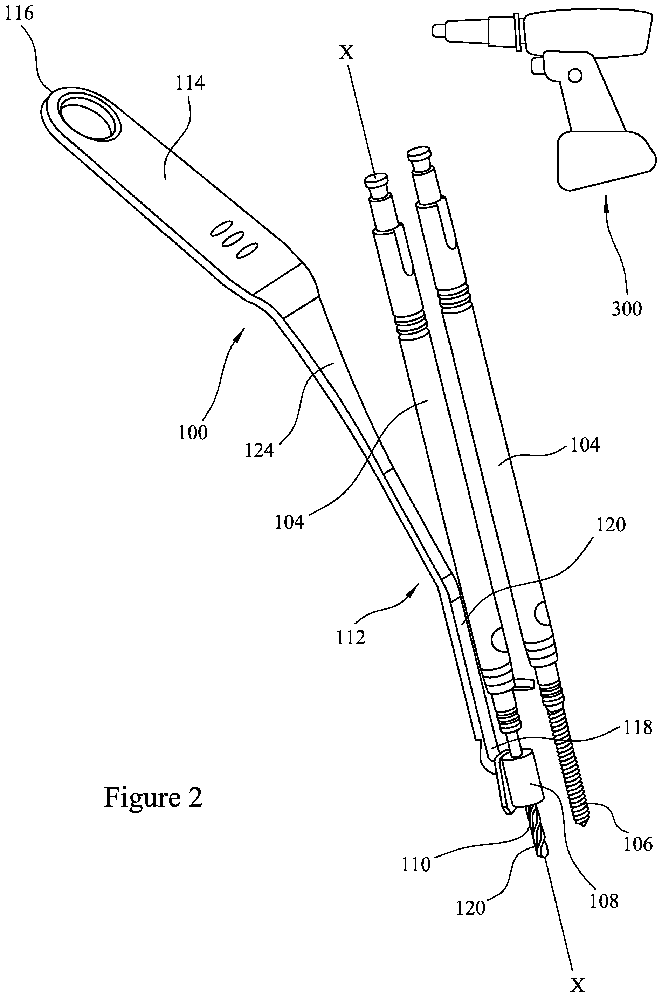

Figure 2 illustrates one example of the disclosure which provides a surgical instrument in the form of guide instrument 100, a surgical tool in the form of a drill bit 102, an intermediate section in the form of an extension drive 104. Figure 2 also shows the same extension drive 104 engaged with a different surgical tool, a self-tapping screw 106.

The guide instrument 100 includes a guide element in the form of a guide piece 108 at the distal end 110. The guide piece 108 is mounted on a stem 112 with a handle portion 114 at the proximal end 116. The stem 112 is divided into several sections. A first section, a transition section 118, spaces the rest of the guide instrument 100 from the operative axis of the drill bit 102. A second section, aligned section 120, is aligned with but spaced from the operative axis of the drill bit 102 and thereby aligns force applied through the handle portion 114 to the liner 25 in use, with the axis along which force applied through the drill bit 102 passes to the liner 25 in use. A third section, inclined section 124, is inclined away from the operative axis of the drill bit 102 so as to increase the space and improve the line of sight for the surgeon down to the distal end 110. The third section, inclined section 124, leads to the handle portion 114.

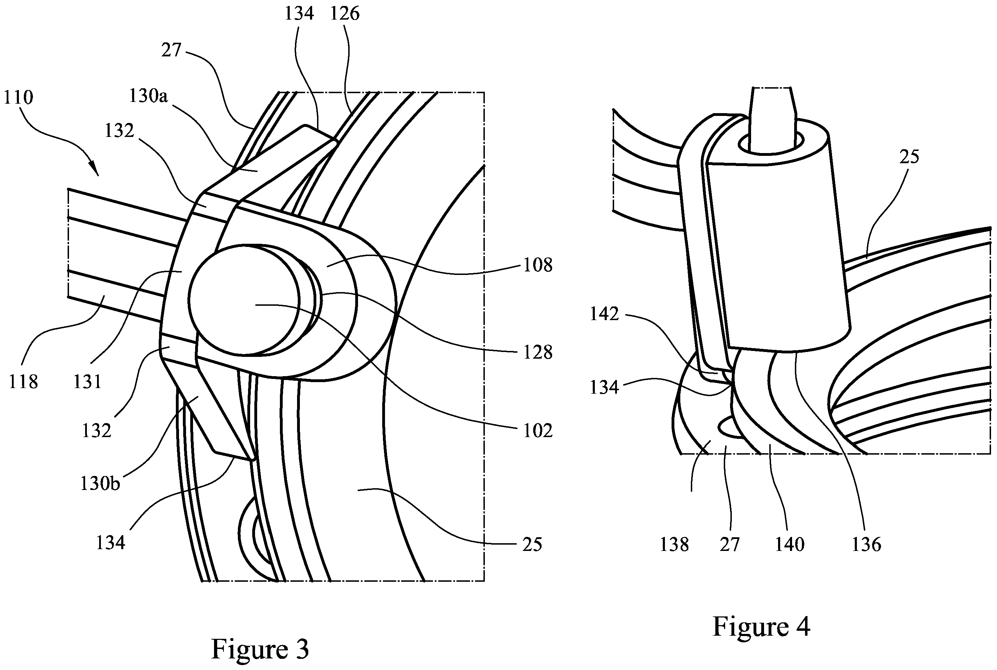

In use, as shown in Figure 3, the distal end 110 of the guide instrument 100 is brought into proximity with the junction 126 between the liner 25 and the shell 27. The guide piece 108 on the distal end 110 provides a guide channel in the form of a through bore 128 through which the drill bit 102 can be inserted. The axis of the bore 128 matches the operative axis for the drill bit 102, in use.

The guide piece 108 is provided with a configuration that provides visual clues and guides to its correct alignment/positioning with respect to a liner 25 and/or shell 27. The guide piece 108 includes a first extending element 130a which extends from a body element 131, provided at the junction of the guide piece 108 and the transition section 118. The first extending element 130a extends in a first direction. A second extending element 130b is provided in the same manner, but extending in the opposing direction. The first and second extending elements are one form of the positioning location supports. Both the first extending element 130a and the second extending element 130b have a body element contacting end 132 and an extending element protruding end 137. The two protruding ends 137 are turned towards one another. A chord drawn between the two protruding ends 137 will pass closer to the axis of the bore 128 than a chord drawn between other parts of the extending elements and particularly between the body element contacting ends 132. In the illustrated example this is provided by curvature of the first extending element 130a and the second extending element 130b.

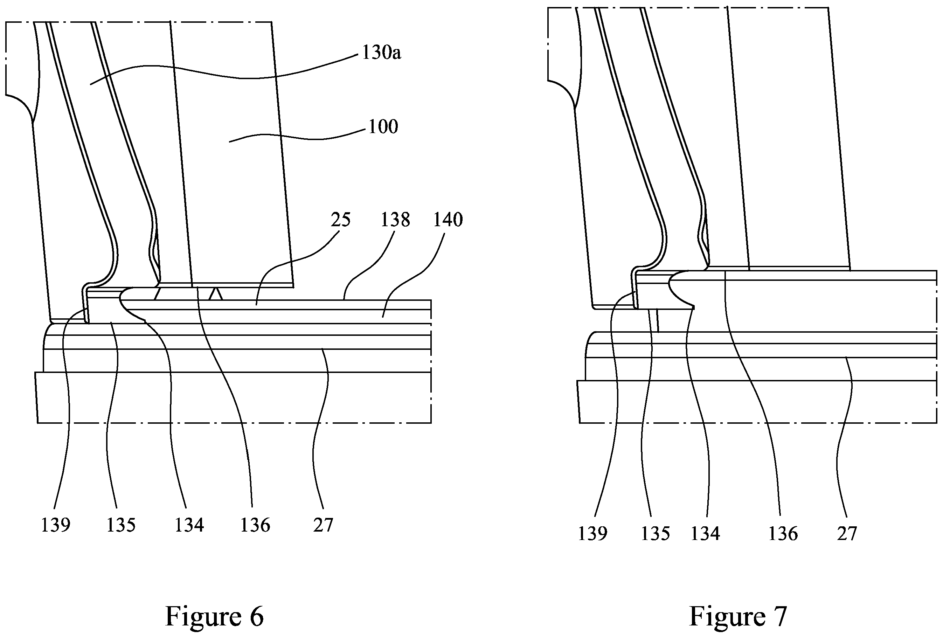

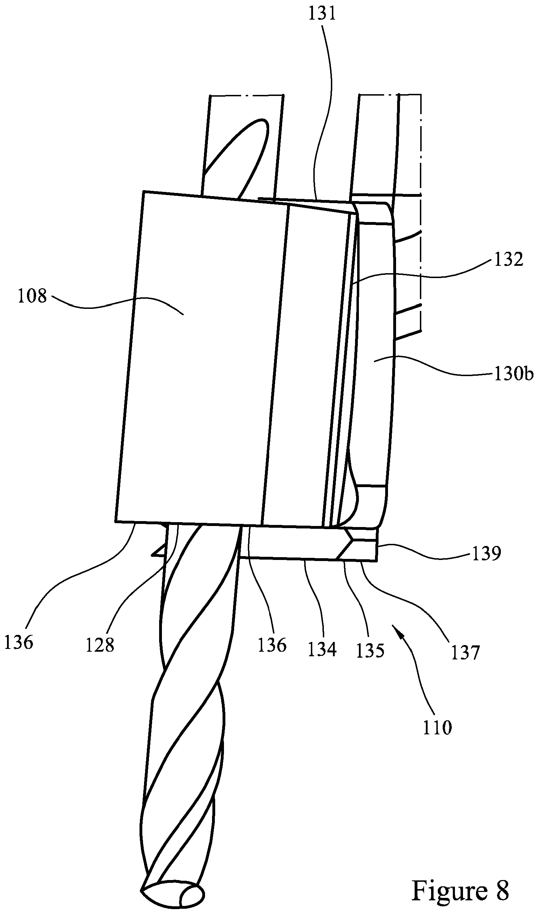

At the distal end 110 of the guide piece 108, as seen in Figures 6, 7 and 8, a planar distal end face 136 is provided around the bore 128. This is one form of the secondary positioning locations. In this example, a common distal end is provided for both the first extending element 130a and the second extending element 130b by a contiguous planar distal end face 135. This distal end face 135 extends the full length of the curvature of both the first extending element 30a and the second extending element 30b. This is one form or the positioning locations. The distal end face 135 is provided on a distal end section 139. The distal end section 139 has a smaller cross-section away from the distal end face 135 than at the distal end face 135 and hence a distal edge 134 is defined. The distal edge 134 extends along the full curvature of both the first extending element 30a and the second extending element 30b in this form.

Referring to Figure 6, the interaction of the distal edge 134 with a first combination of shell 27 and liner 25 is shown. In this example, the liner 25 has a relatively small extent of protrusion from the shell 27 and so the perimeter edge wall 140 has only a small height. Different interactions, as further exemplified below, will occur for different liner 25 and shell 27 combinations.

In Figure 6, the distal end face 135 and distal edge 134 has a greater distal extent relative to the guide instrument 100 than the guide piece 108, for instance the distal end face 136 thereof. The planar distal end face 135 is parallel to but not co-planar with the distal end face 136 of the guide piece 108.

In use, this means that as the guide instrument 100 is brought towards the shell 27 and liner 25, one end then the other or both simultaneously of the distal end face 135 will abut the circumferential end face 138 of the shell 27. This limits generally axial movement of the guide instrument 100. The distal end face 135 can then be slid radially inward relative to the shell 27 and liner 25 across the end face 138. This continues until parts of the distal edge 134 abut the raised perimeter edge wall 140 of the liner 25 formed by the liner 25 extending slightly above the plane of the end face 138 of the shell 27. At this point, the liner 25 resists further inward movement. In the illustrated form, the curvature of the distal edge 134 is such that the ends of the distal edge 134 provide the abutment. The profile of the distal edge 134 is such that its ends act like teeth 142. The distal edge 134 faces laterally away from the extending elements relative to the proximal to distal orientation of those extending elements. That is the distal edge 134 faces towards the liner 25 in use, and in particular towards the perimeter edge wall 140 at the junction of the liner 25 and shell 27. The application of radial force to the guide piece 108 through the handle portion 114 causes the teeth 142 at the ends of the distal edge 134 to penetrate the material of the liner 25 and particularly to penetrate the perimeter edge wall 140. The limited extent of the distal edge 134 and it not being possible to cause the extending element behind them to penetrate the liner 25, means that the radial position of the extending elements 130a, 130b and hence of the guide piece 108 and hence the bore 128 is very closely controlled. The pilot hole is created in a consistent and precise location.

In an alternative format, the curvature of the distal edge 134, particularly its radius of curvature, could be greater than the curvature, particularly radius of curvature of the liner, such that intermediate locations on the distal edge 134 abut the liner. The distal edge 134 is sharp enough along its length to penetrate the liner 25 and so act in a tooth like manner at that abutment.

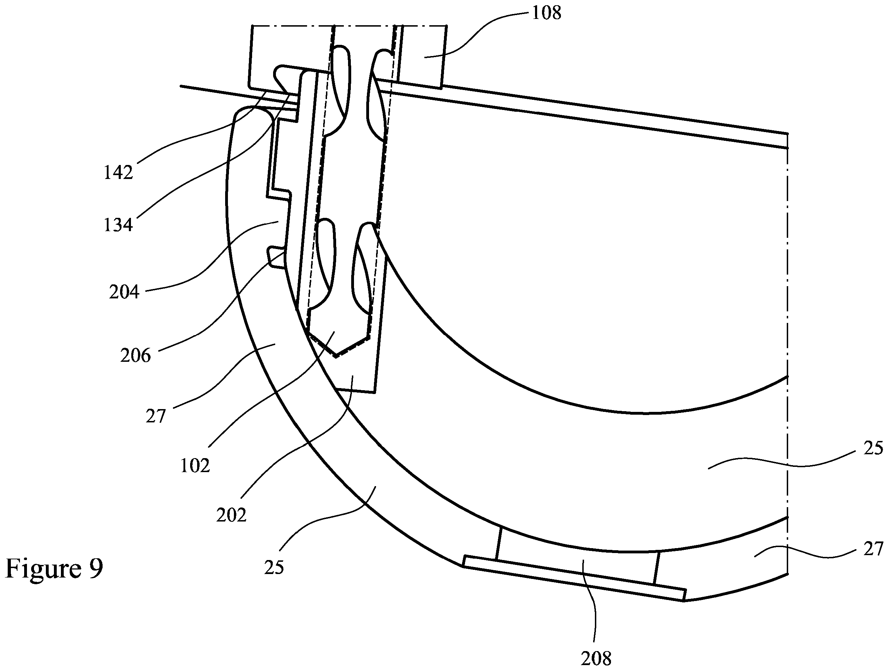

Figure 9 shows the pilot hole 202 being formed by a drill bit 102. The drill bit 102 extends through the guide piece 108, into the liner 25 and down to the junction between the liner 25 and the shell 27. The teeth 142 at the ends of the curved edge 134 are engaged with the liner 25.

The location used for the pilot hole 202 is adjacent the peripheral edge of the liner 25 and is also adjacent the peripheral edge of the shell 27. In this area, no contact between the shell 27 and the liner 25 is intended in use, and so, even if the removal of the liner 25 by the disclosure causes minor damage to the shell 27, that minor damage is not in a material location on the shell 27. If a location further into the liner 25, for instance at or close to the pole of the liner 25 and shell 27, was used then any damage would be in a material area. The location used for the pilot hole 202 is however far enough radially inward relative to the edge of the liner 25, that the pilot hole 202 is away from the retaining mechanism 204, 206 for the liner 25 in the shell 27. In the illustrated example, a lug 204 on the shell 27 engages with a recess 206 on the liner 25, but other retention mechanisms are possible.

The peripheral location for the pilot hole 202 has been established in testing to offer the most effective approach to removing the liner 25 from the shell. This is particularly important in providing enough force to overcome the retention force between the liner 25 and the shell 27. The peripheral location is more effective than locations further into the liner 25, for instance at or close to the pole 208 of the liner 25 and shell 27.

Referring to Figure 7, a different interaction of the distal edge 134 with a first combination of shell 27 and liner 25 is shown. In this example, the liner 25 has a relatively large extent of protrusion from the shell 27 and so the perimeter edge wall 140 has a significant height.

In use, this means that as the guide instrument 100 is brought towards the shell 27 and liner 25, the distal end face 136 will abut the circumferential end face 200 of the liner 25. This limits generally axial movement of the guide instrument 100. The distal edge 134 in this position is still spaced from the circumferential end face 138 of the shell 27, but the distal edge 134 can still be slid radially inward relative to the shell 27 and liner 25 until sections of the distal edge 134, in the illustrated example the teeth 142 at the ends, abut the raised perimeter edge wall 140 of the liner 25 formed by the liner 25 extending slightly above the plane of the end face 138 of the shell 27. At this point, the liner 25 resists further inward movement. The application of radial force to the guide piece 108 through the handle portion 114 causes the distal edge to penetrate the material of the liner 25 and particularly to penetrate the perimeter edge wall 140.

Whilst the embodiments above reference a tooth 142 at each end of a continuous distal edge 134 for providing the penetration of the liner 25, it is possible to provide two or more teeth in other positions. A series of teeth spaced along the distal edge 134 and/or a serrated distal edge 134 could be provided. Equally it is also possible to rely upon intermediate parts of the distal edge 134 engaging with and penetrating the liner 25.

The configuration of the distal edge 134 of the extending elements and/or the teeth 142 means that a wide range of different diameter shells 27 and liners 25 can be used successfully with the same guide instrument 100.

The drill bit 102 is provided separately from the guide instrument 100 and is mounted on an extension drive or other actuating instrument 104, optionally in the manner described in more detail below. The same diameter of drill bit 102 is suitable for a wide range of liner 25 sizes and so a given guide piece 108 and guide instrument 100 is also widely applicable.

The guide piece 108 through bore 128 has an axis X-X which gives the desired pilot hole with a consistent angularity with respect to the liner 25 as the bore 28 limits the operative axis of the drill bit 102 to the same axis X-X. The cross-section of the bore 28 perpendicular to the axis X-X [plus a clearance tolerance] matches that of the cross-section of the drill bit 102 also perpendicular to the axis X-X.

In use, therefore, with the guide instrument 100 in position on the shell 27 and liner 25, with parts of the distal edge 134 or teeth 142 in the material of the liner 25, the drill bit 102 on the drive extension 104 is slid into the bore 128 and into abutment with the surface of the liner 25. A manual or powered actuator 300 [shown schematically in Figure 2] connected to the drive extension 104 is used to rotate the drill bit 102 and hence form a pilot hole in the liner 25. Once the desired depth of pilot hole is formed, the drive extension 104 and the drill bit 102 can be retracted. The guide instrument 100 can then be moved outward to disengage the parts of the distal edge 134 or teeth 142 and can then also be withdrawn.

If necessary, the process can be repeated at other locations to provide other pilot holes.

A drive extension 104, which could be the same one with the drill bit 102 disconnected, is then provided with a self-tapping screw 106. The distal end of the screw 106 is inserted into the end of the pilot hole and rotation of the drive extension 104 causes the screw 106 to advance into the liner 25. Continued advancement of the screw 106 causes the liner 25 to be displaced from the shell 27.

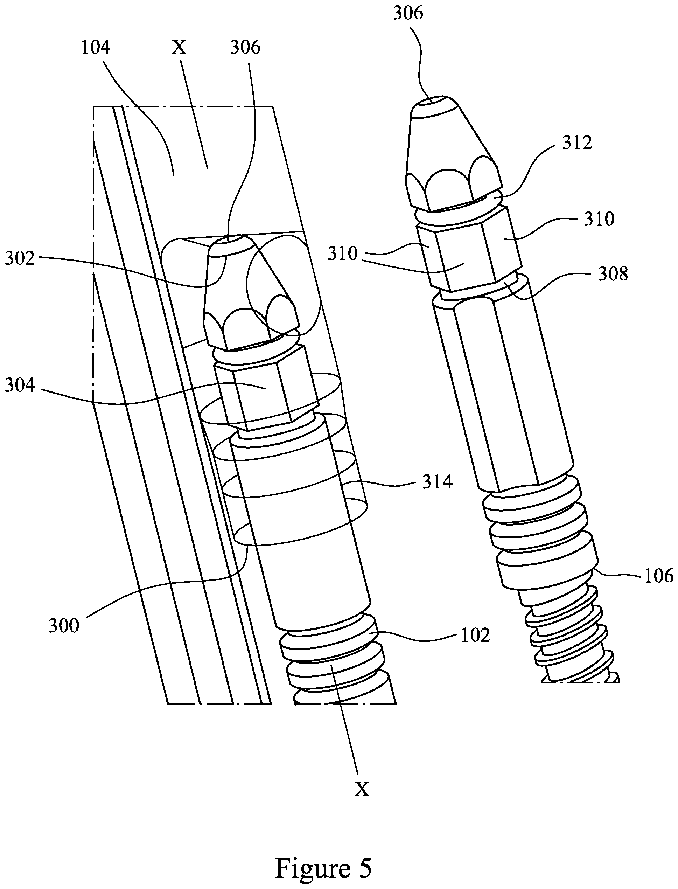

The drill bit 102 can be mounted on an extension drive 104 or other actuating instrument 104 in various ways. One option under the disclosure is illustrated in Figure 5.

The drive extension 104 has an internal bore in its distal end 300 which receives the proximal end 302 and end section 304 of the drill bit 102 and similarly the proximal end 306 and end section 308 of the screw 106. The end sections 304, 308 have equivalent cross-sectional profiles and as illustrated are provided with six regularly spaced and matching size flats 310 which provide an hexagonal drive interface. The internal bore of the drive extension 104 is provided with a corresponding drive profile; in the illustrated case with six regularly spaced and matching size flats 310 which provide an hexagonal drive interface. The cooperation of the two drive faces readily transfers torque from the drive extension 104 to the drill bit 102 or screw 106. The depth of the internal bore, considered along axis X-X, matches the length of the end sections 304, 308 such that abutment of the proximal ends 302, 306 with the base 312 of the bore 314 results in correct alignment of the drive interfaces. The abutment also constrains axial movement of the drill bit 102 or screw 106 into the drive extension 104 and allows application of axial force to encourage the drill bit 102 or screw 106 into the material of the liner 25.

It is desirable to provide for easy engagement and disengagement of the drill bit 102 and/or screw 106 with the drive extension 104. At the same time, there is a need to retain the drill bit 102 and/or screw 106 on the drive extension 104 during their movement to the position of use and during their movement away from the position of use. In the disclosure, this level of axial restraint for the drill bit 102 and/or screw 106 against relative axial movement away from the drive extension 104 is provided by the interaction of an O-ring 312 on one with the surface on the other. In one form, the O-ring 312 is provided on the single use drill bit 102 rather than in the internal bore 314 of the drive extension 104. Similarly the O-ring 312 may be provided on the single use screw 106 rather than in the internal bore 314 of the drive extension 104.

In that one form, as the drill bit 102 is slid into the internal bore 314, at least when the proximal end 302 approaches the base 312 of the bore 314, the internal profile of the bore 314 causes compression of the O-ring 312. The resilience of the O-ring 312 resists this compression and hence resists axial movement of the drill bit 102 out of the bore 314. The resistance is sufficient to prevent the weight of the drill bit 102 or a knock to the drill bit 102 causing it to detach from the drive extension 104. The O-ring 312 on the screw 106 operates and interacts in an equivalent manner. The resistance to axial movement is limited, however, and so the drill bit 102 or screw 106 can readily be pulled out of the drive extension 104 when desired, for instance to swap from the drill bit 102 to a screw 106. As the drill bit 102 and screw 106 are intended to be single use, there is no need to sterilise a structure with an O-ring 312 present.

While the concepts of the present disclosure are susceptible to various modifications and alternative forms, specific embodiments thereof have been shown by way of example in the drawings and will be described herein in detail. It should be understood, however, that there is no intent to limit the concepts of the present disclosure to the particular forms disclosed, but on the contrary, the intention is to cover all modifications and alternatives within the scope of the invention as defined by the appended claims.

## Claims

1. A surgical instrument (100), the instrument comprising:
a stem (112), the stem having a proximal end (116) and having a distal end (110);
a handle portion (114), the handle portion being provided towards the proximal end of the stem; and
a guide element (108), the guide element being provided towards the distal end of the stem, wherein the guide element provides:
a body element defining a guide channel (128), the guide channel having a longitudinal axis and being open at both ends along the longitudinal axis; and
one or more positioning elements (130a, 130b), **characterised in that** a curved element and/or an edge (134) is provided towards the distal end of the one or more positioning elements, and wherein the curved element and/or edge project away from the guide element towards a longitudinal axis of the guide channel.

2. The instrument of claim 1, wherein the guide channel is adapted to receive a cutting tool (102) and the longitudinal axis of the guide channel corresponds to the operative axis of the cutting tool in use.

3. The instrument of claim 1 or claim 2, wherein the guide channel is adapted to restrain radial movement of the cutting tool and allow axial movement of the cutting tool relative to the guide channel.

4. The instrument of any preceding claim, wherein the one or more positioning elements provide liner penetrating parts (142).

5. The instrument of any preceding claim, wherein two or more positioning elements are provided and wherein each positioning element provides a liner penetrating part (142) towards its distal end.

6. The instrument of any preceding claim, wherein the curved element and/or edge provide, in use, the liner penetrating parts.

7. A system, the surgical system comprising:
the surgical instrument according to claim 1;
the system further comprising:
one or more cutting tools (102); and
one or more elongate at least partially threaded elements (106).

8. A system according to claim 7, wherein the surgical systems further comprises one or more of:
an actuator (300) for application of torque to the cutting tool;
one or more drill bits;
one or more self-tapping screw threads.

9. A system according to claim 7 or claim 8, wherein the guide channel of the surgical instrument is configured to receive the cutting tool with the cross-section of the guide channel being the cross-section of the cutting tool plus a clearance.

10. The system of any of claims 7 to 9, wherein one or more or all of the positioning elements provide one or more liner penetrating parts (142).

11. The system of any of claims 7 to 10, wherein two or more positioning elements are provided and wherein each positioning element provides a liner penetrating part (142) towards its distal end.

## Patentansprüche

1. Chirurgisches Instrument (100), wobei das Instrument umfasst:
einen Schaft (112), wobei der Schaft ein proximales Ende (116) und ein distales Ende (110) aufweist;
einen Griffabschnitt (114), wobei der Griffabschnitt zu dem proximalen Ende des Schafts hin bereitgestellt ist; und
ein Führungselement (108), wobei das Führungselement zu dem distalen Ende des Schafts hin bereitgestellt ist, wobei das Führungselement bereitstellt:
ein Körperelement, das einen Führungskanal (128) definiert, wobei der Führungskanal eine Längsachse aufweist und an beiden Enden entlang der Längsachse offen ist; und
ein oder mehrere Positionierungselemente (130a, 130b), **dadurch gekennzeichnet, dass** ein gekrümmtes Element und/oder eine Kante bzw. ein Rand (134) zu dem distalen Ende des einen oder der mehreren Positionierungselemente hin bereitgestellt ist, und wobei das gekrümmte Element und/oder die Kante von dem Führungselement weg zu einer Längsachse des Führungskanals hin vorstehen.

2. Instrument nach Anspruch 1, wobei der Führungskanal angepasst ist, ein Schneidwerkzeug (102) aufzunehmen, und die Längsachse des Führungskanals der operativen Achse des Schneidwerkzeugs in Gebrauch entspricht.

3. Instrument nach Anspruch 1 oder Anspruch 2, wobei der Führungskanal angepasst ist, eine radiale Bewegung des Schneidwerkzeugs einzuschränken und eine axiale Bewegung des Schneidwerkzeugs relativ zu dem Führungskanal zu ermöglichen.

4. Instrument nach einem der vorhergehenden Ansprüche, wobei das eine oder die mehreren Positionierungselemente Liner-Durchdringungsteile (142) bereitstellen.

5. Instrument nach einem der vorhergehenden Ansprüche, wobei zwei oder mehr Positionierungselemente bereitgestellt sind und wobei jedes Positionierungselement ein Liner-Durchdringungsteil (142) zu seinem distalen Ende hin bereitstellt.

6. Instrument nach einem der vorhergehenden Ansprüche, wobei das gekrümmte Element und/oder die Kante in Gebrauch die Liner-Durchdringungsteile bereitstellen.

7. System, wobei das chirurgische System umfasst:
das chirurgische Instrument nach Anspruch 1;
wobei das System ferner umfasst:
ein oder mehrere Schneidwerkzeuge (102); und
ein oder mehrere längliche, zumindest teilweise mit Gewinde versehene Elemente (106).

8. System nach Anspruch 7, wobei die chirurgischen Systeme ferner eines oder mehrere umfassen:
einen Aktor (300) zur Aufbringung von Drehmoment auf das Schneidwerkzeug;
einen oder mehrere Bohrer bzw. Bohrspitzen;
ein oder mehrere selbstschneidende Schraubengewinde.

9. System nach Anspruch 7 oder Anspruch 8, wobei der Führungskanal des chirurgischen Instruments dahingehend konfiguriert ist, das Schneidwerkzeug aufzunehmen, wobei der Querschnitt des Führungskanals der Querschnitt des Schneidwerkzeugs plus ein Zwischenraum ist.

10. System nach einem der Ansprüche 7 bis 9, wobei ein oder mehrere oder alle der Positionierungselemente ein oder mehrere Liner-Durchdringungsteile (142) bereitstellen.

11. System nach einem der Ansprüche 7 bis 10, wobei zwei oder mehr Positionierungselemente bereitgestellt sind und wobei jedes Positionierungselement ein Liner-Durchdringungsteil (142) zu seinem distalen Ende hin bereitstellt.

## Revendications

1. Instrument chirurgical (100), l'instrument comprenant :
une tige (112), la tige ayant une extrémité proximale (116) et ayant une extrémité distale (110) ;
une partie de poignée (114), la partie de poignée étant prévue vers l'extrémité proximale de la tige ; et
un élément de guidage (108), l'élément de guidage étant prévu vers l'extrémité distale de la tige, dans lequel l'élément de guidage fournit :
un élément de corps définissant un canal de guidage (128), le canal de guidage ayant un axe longitudinal et étant ouvert aux deux extrémités le long de l'axe longitudinal ; et
un ou plusieurs éléments de positionnement (130a, 130b), **caractérisé en ce que** :
un élément courbé et/ou un bord (134) est prévu vers l'extrémité distale des un ou plusieurs éléments de positionnement, et
dans lequel l'élément courbé et/ou le bord font saillie à l'opposé de l'élément de guidage vers un axe longitudinal du canal de guidage.

2. Instrument selon la revendication 1, dans lequel le canal de guidage est adapté pour recevoir un outil de coupe (102) et l'axe longitudinal du canal de guidage correspond à l'axe opérationnel de l'outil de coupe, à l'usage.

3. Instrument selon la revendication 1 ou la revendication 2, dans lequel le canal de guidage est adapté pour limiter le mouvement radial de l'outil de coupe et permettre le mouvement axial de l'outil de coupe par rapport au canal de guidage.

4. Instrument selon l'une quelconque des revendications précédentes, dans lequel les un ou plusieurs éléments de positionnement fournissent des parties de pénétration de revêtement (142).

5. Instrument selon l'une quelconque des revendications précédentes, dans lequel deux éléments de positionnement ou plus sont prévus et dans lequel chaque élément de positionnement fournit une partie de pénétration de revêtement (142) vers son extrémité distale.

6. Instrument selon l'une quelconque des revendications précédentes, dans lequel l'élément courbé et/ou le bord fournissent, à l'usage, les parties de pénétration de revêtement.

7. Système, le système chirurgical comprenant :
l'instrument chirurgical selon la revendication 1 ;
le système comprenant en outre :
un ou plusieurs outils de coupe (102) ; et
un ou plusieurs éléments allongés au moins partiellement filetés (106).

8. Système selon la revendication 7, dans lequel les systèmes chirurgicaux comprennent en outre un ou plusieurs parmi :
un actionneur (300) pour l'application de couple sur l'outil de coupe ;
un ou plusieurs forets ;
un ou plusieurs filetages de vis autotaraudeuse.

9. Système selon la revendication 7 ou la revendication 8, dans lequel le canal de guidage de l'instrument chirurgical est configuré pour recevoir l'outil de coupe avec la section transversale du canal de guidage qui est la section transversale de l'outil de coupe plus un jeu.

10. Système selon l'une quelconque des revendications 7 à 9, dans lequel un ou plusieurs ou la totalité des éléments de positionnement fournissent une ou plusieurs parties de pénétration de revêtement (142).

11. Système selon l'une quelconque des revendications 7 à 10, dans lequel deux éléments de positionnement ou plus sont prévus et dans lequel chaque élément de positionnement fournit une partie de pénétration de revêtement (142) vers son extrémité distale.
